# EUROPÄISCHE PATENTSCHRIFT

(11) **EP 0 713 913 B1**
(45) Veröffentlichungstag und Bekanntmachung des Hinweises auf die Patenterteilung: **26.03.2003**
(21) Anmeldenummer: 95118179.1
(22) Anmeldetag: 18.11.1995
(51) Int. Cl.: C12N 15/10, C12Q 1/68

(54) **Verbessertes Transkriptionsverfahren**
Improved transcription assay
Système de transcription amélioré

(30) Priorität: 23.11.1994 DE 4441602
(43) Veröffentlichungstag der Anmeldung: 29.05.1996
(73) Patentinhaber: Roche Diagnostics GmbH, 68298 Mannheim (DE)
(72) Erfinder: Rosemeyer, Viola, B-1300 Wavre (BE); Seibl, Rudolf, Dr., D-82377 Penzberg (DE)

(56) Entgegenhaltungen:
- EP-A- 0 369 775
- WO-A-91/01384
- WO-A-92/18521

## Beschreibung

Gegenstand der vorliegenden Erfindung sind Verfahren zur Transkription einer Templat-Nukleinsäuresequenz in einer Templat-Nukleinsäure in Ribonukleinsäure, hierfür geeigneter Reagenzien und vorteilhafte Verwendung dieser Reagenzien.

Die in-vitro-Transkription ist ein etabliertes Verfahren zur Erzeugung von RNA-Molekülen. Dafür werden neben der gewünschten Matrizensequenz, die im folgenden Templat-Nukleinsäuresequenz genannt wird, eine RNA-Polymerase sowie ein mit der Templat-Nukleinsäuresequenz funktionell verbundener Promotor benötigt. Vorzugsweise werden RNA-Polymerasen der Phagen T3, T7 oder SP6 verwendet, da sie einfach zu handhaben sind und eine hohe Transkriptionseffizienz aufweisen. Als Matrizenmoleküle, die im Folgenden Templat-Nukleinsäuren genannt werden, werden sowohl Polynukleotide als auch Oligonukleotide verwendet. Polynukleotide können zum Beispiel über PCR erzeugt werden oder in entsprechenden Transkriptionsvektoren kloniert vorliegen. Auch die Verwendung von Oligonukleotiden, das heißt kurzen Nukleinsäuren, zur Generierung von RNA-Oligonukleotiden ist bekannt.

In EP-A-0408295 ist ein Verfahren zur Amplifikation von Nukleinsäure vorgeschlagen, bei dem eine partiell doppelsträngige Nukleinsäure einer Transkriptionsreaktion unterworfen wird, wobei die Nukleinsäure aus einer DNA-Zielsequenz und einer Promotornukleinsäure besteht, wobei die Promotornukleinsäure aus einer Promotorsequenz, einer zu der Ziel-DNA komplementären Sequenz und einer zum Gegenstrang der Ziel-DNA komplementären Sequenz besteht. Aus den Reaktionsbedingungen läßt sich schließen, daß die Promotornukleinsäure aus DNA besteht. Anderenfalls würde ein Abbau der Promotornukleinsäure durch die im Reaktionsgemisch enthaltene RNAse stattfinden.

Aus J. Mol. Biol. 224, 319-334 (1992) ist bekannt, daß bei in-vitro-Transkriptionsverfahren in sehr großer Menge sogenannte abortive Transkripte mit einer Länge von bis zu etwa 9 Nukleotiden entstehen. Die kurzen Transkripte lassen sich nicht quantitativ durch Standardverfahren, wie zum Beipiel die Präzipitation mittels Ethanol, abtrennen. Es sind weitaus aufwendigere Verfahren, wie Reinigung mittels HPLC oder Gelelektrophorese, notwendig. Die abortiven Transkripte verhindern die exakte Bestimmung der Menge an erhaltenen Transkripten voller Länge (z. B. durch photometrische Messungen). Des weiteren wird durch die Entstehung der abortiven Transkripte die Ausbeute an Transkripten voller Länge vermindert.

Aufgabe der vorliegenden Erfindung war es, die herkömmlichen Transkriptionsverfahren zu verbessern, insbesondere die Bildung abortiver Transkripte zu reduzieren.

Gegenstand der Erfindung ist daher ein Verfahren zur Transkription einer Templat-Nukleinsäuresequenz TN in einer Templat-Nukleinsäure T in Ribonukleinsäure R durch Bildung eines partiell oder vollständig doppelsträngigen Nukleinsäurekomplexes, enthaltend eine funktionelle, zumindest partiell doppelsträngige, Promotorregion aus Promotorsequenzen PP und PP', eine Effektorsequenz PE und eine mit der Promotorregion funktionell verknüpfte Templat-Nukleinsäuresequenz TN und Einstellung von Bedingungen, unter denen die Ribonukleinsäure R unter Verwendung von TN als Templat-Sequenz unter Kontrolle des Promotors gebildet wird.

Kern der Erfindung ist die Erkenntnis, daß die Menge an entstehenden abortiven Transkripten durch Anwesenheit einer Effektorsequenz PE (z. B. in der oder an die Promotorregion) verringert wird ohne die Ausbeute an Transkripten voller Länge allzu negativ zu beeinflussen. Diese Erkenntnis kann prinzipiell auf alle Transkriptionsverfahren angewandt werden, wenn sie nur unter der Kontrolle eines Promotors ablaufen. Besonders bedeutsam dürfte die Erkenntnis für in-vitro-Transkriptionen sowie für Transkriptionen unter Verwendung von Phagen-RNA-Polymerasen wie T7, T3 oder SP6 sein.

Unter Nukleinsäuren werden im Folgenden sowohl die natürlichen als auch künstliche Nukleinsäuren verstanden. Natürliche Nukleinsäuren sind solche, wie sie beispielsweise in Viren, Bakterien oder mehrzelligen Organismen vorkommen. Sie enthalten, an einem Zuckerphosphatgrundgerüst, Nukleobasen, deren Aufeinanderfolge an dem Grundgerüst für den betreffenden Organismus oder Teile seines Genoms charakteristisch ist. Ein wesentliches Merkmal von Nukleinsäuren ist die Fähigkeit zur Bildung von Doppelsträngen mit Nukleinsäuren, die eine hierzu komplementäre Nukleobasensequenz aufweisen.

Unter künstlichen Nukleinsäuren werden solche verstanden, die in ihrem Aufbau modifiziert sind, z. B. durch Ersatz des Zuckerphosphatgrundgerüstes durch ein Polypeptidgrundgerüst oder durch Einführung nicht natürlich vorkommender Basen anstelle der natürlichen Basen, die jedoch noch die Fähigkeit zur Bildung von Doppelsträngen mit anderen Nukleinsäuren, insbesondere den natürlichen Nukleinsäuren, aufweisen.

Unter Transkription wird der in der Fachwelt bekannte Vorgang der Erzeugung von Ribonukleinsäuren mit Verwendung einer Templat-Nukleinsäure unter der Kontrolle eines Promotors durch eine RNA-Polymerase verstanden.

Unter einer Templat-Nukleinsäure T wird eine einzel- oder doppelsträngige oder partiell doppelsträngige Nukleinsäure verstanden, die eine Templat-Nukleinsäuresequenz TN enthält, die in Ribonukleinsäure (R) umgeschrieben werden soll. Die entstehende Ribonukleinsäure ist komplementär zu der Templat-Nukleinsäuresequenz. Daher wird der die Templat-Nukleinsäuresequenz TN enthaltende Strang oft als codierender Strang bezeichnet. Der codierende Strang muß eine Deoxyribonukleinsäure (DNA) sein.

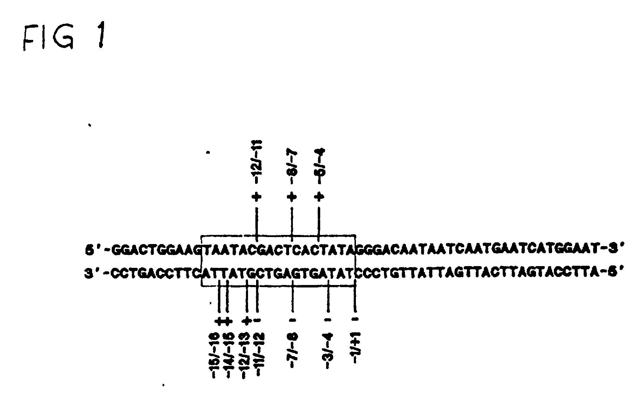

Ein Promotor im Sinne der vorliegenden Erfindung ist eine mindestens partiell doppelsträngige Nukleinsäureregion als Bindungsstelle für eine RNA Polymerase und Initiationsstelle für die Transkription. Wenn eine Templat-Nukleinsäure an das 5'-Ende seines codierenden Stranges angehängt ist, werden unter geeigneten Reaktionsbedingungen Transkripte der Templat-Nukleinsäuresequenz synthetisiert. Dieser Promotor wird dann als funktionell mit der Templat-Nukleinsäuresequenz verknüpft bezeichnet. Gemäß der vorliegenden Erfindung kann ein Teil eines Promotorstranges fehlen. Wichtig erscheint, daß die für die Funktionalität des Promotors erforderliche Gesamtlänge erhalten bleibt, sei es partiell oder vollständig doppelsträngig. Beispielhaft wurde gezeigt, daß die Einführung sogenannter Nicks in (dem Doppelstrang) der Promotorregion in vielen Fällen keinen oder nur unbedeutenden Einfluß auf die Transkriptionseffizienz hat. Für den Fall des T7-Promotors wurde gezeigt, daß Nicks zwischen Positionen -5/-4, -8/-7, -12/-11 des nicht-codierenden Strangs sowie den Positionen -12/-13, -14/-15 und -15/-16 des T7-Promotors möglich sind (FIG 1). Weiterhin erscheint es wichtig, daß der nicht-codierende Strang PP des Promotors vom 5'-Ende her nur ungefähr bis Position -8 reichen muß, während der codierende Strang vom 5'-Ende her mindestens bis ungefähr zur Position -13 reichen muß, ohne daß der folgende einzelsträngige Bereich durch Hybridisierung mit einer weiteren Nukleinsäure doppelsträngig gemacht werden muß.

Der codierende Strang des Promotors wird im folgenden als Promotorsequenz PP' und der nicht codierende Strang als Promotorsequenz PP bezeichnet. Die Aufeinanderfolge der Nukleobasen in der Promotorregion ist für eine Reihe von Promotoren aus Phagen, z. B. T3, T7 oder SP6, bekannt.

Als Sequenz PT wird eine mit der Templat-Nukleinsäure hybridisierbare Sequenz bezeichnet. Sequenz PT ist daher im wesentlichen komplementär zu einem Teil der Templat-Nukleinsäure. Bevorzugt schließt sich an diesen Teil der Templat-Nukleinsäure stromabwärts des Promotors eine ausgedehnte Templat-Nukleinsäuresequenz an, die, beginnend mit dem Teil, zu dem die Sequenz PT hybridisieren kann, Gegenstand der Transkriptionsreaktion ist. Bei der Sequenz PT handelt es sich bevorzugt nicht um RNA.

Bei der erfindungsgemäßen Effektorsequenz handelt es sich ebenfalls um eine Nukleinsäure. In einem ersten bevorzugten Aspekt der Erfindung ist die Aufeinanderfolge der Nukleobasen in der Effektorsequenz dergestalt, daß unter den gewählten Bedingungen möglichst keine Hybridisierung der Effektorsequenz mit der Templat-Nukleinsäuresequenz TN, deren Gegenstrang TN', der Promotorsequenz PP oder deren Gegenstrang PP' stattfinden kann. Bevorzugt sollte die Effektorsequenz auch mit weiteren in der Reaktionsmischung vorhandenen Nukleinsäuren nicht hybridisieren. In einem zweiten Aspekt der Erfindung handelt es sich bei der Effektorsequenz bevorzugt um RNA, da die Gesamteffektivität der Transkriptionsreaktion leidet, wenn die Effektorsequenz aus DNA besteht.

Die Länge der Effektorsequenz PE ist weitgehend unwesentlich. Es hat sich jedoch gezeigt, daß sie mindestens 4 Nukleotide lang sein sollte. Bevorzugt ist PE 5 - 50, besonders bevorzugt 10 - 30 Nukleotide lang.

Unter einer Promotornukleinsäure P wird eine Nukleinsäure verstanden, die, neben anderen Nukleinsäuresequenzen, entweder eine Promotorsequenz PP oder/und deren Gegenstrang PP' enthält.

Unter einem Linker wird ein Molekülteil verstanden, welcher ein oder mehrere Atome enthält und zwei Nukleinsäuresequenzen miteinander kovalent verbindet. Der Linker kann nicht-nukleosidisch, jedoch auch nukleosidisch sein. Bevorzugt wird unter einem Linker eine Nukleinsäuresequenz verstanden. In einer Ausführungsform ist der Linker eine mit der Templat-Nukleinsäuresequenz hybridisierbare Sequenz PT.

In einer anderen Ausführungsform ist der Linker eine beliebige Verbindung zwischen einem Promotorstrang und der Effektorsequenz.

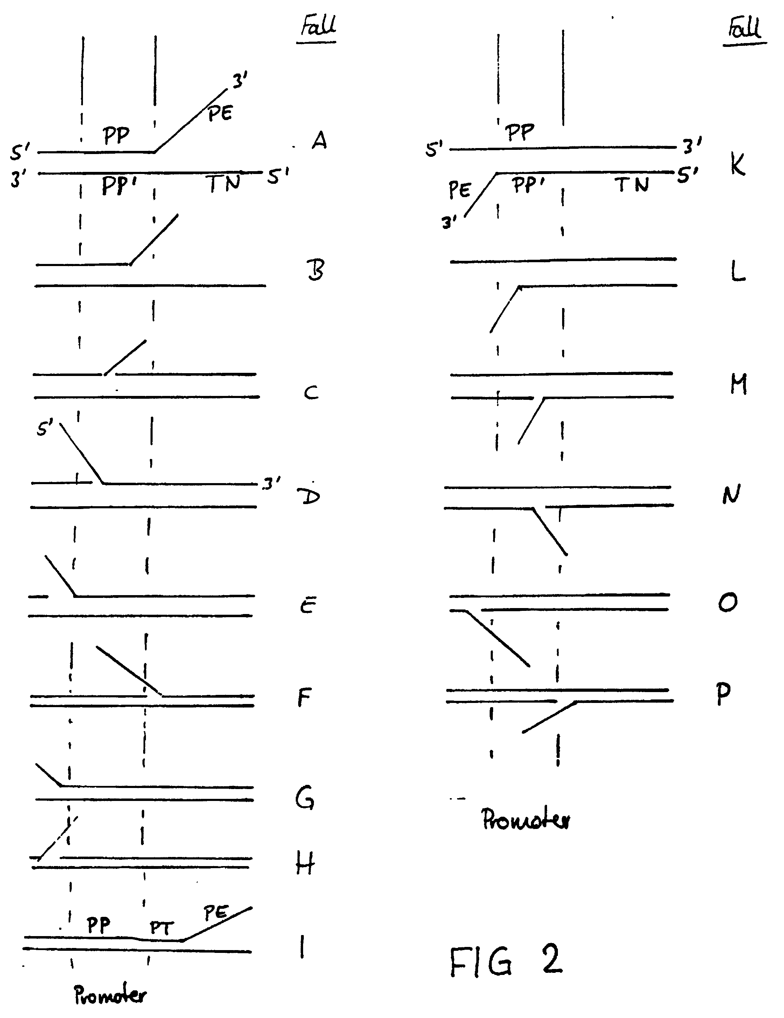

In FIG 2 ist eine Modellnukleinsäure (doppelsträngig, SEQ.ID.NO. 10) gezeigt mit der Toleranz für Nicks.

Bei der Verbindung der Effektorsequenz mit einer Promotorsequenz lassen sich mehrere Fallgestaltungen unterscheiden. Einige dieser Fallgestaltungen sind in Figur 2 abgebildet. In einer ersten Anknüpfungsmöglichkeit ist die Effektorsequenz PE direkt oder über einen Linker an das 3'-Ende der Promotorsequenz PP geknüpft.

In einer zweiten Fallgestaltung (Fall A von FIG 2) ist die Effektorsequenz direkt an das Nukleotid in Position -1 der Promotorsequenz PP geknüpft. Dies kann auf einfache Weise über eine übliche Phosphodiester-Bindung zwischen einer 3'-Hydroxylgruppe der Promotorsequenz und einer 5'-Hydroxylgruppe der Effektorsequenz geschehen.

In einer dritten Ausführungsmöglichkeit (Fall B von FIG 2) ist die Effektorsequenz direkt mit einer 3'-Hydroxylgruppe einer Promotorsequenz PP verknüpft, die gegenüber der vollständigen Promotorsequenz von Position -1 bis zu Position -8 verkürzt sein kann (für den Fall des T7-Promotors). In diesem Fall ist die Benutzung eines zweiten Moleküles welches die nicht in PP enthaltenen Nukleotide des Promotors enthält, nicht erforderlich.

In einer vierten Ausführungsform ist die Effektorsequenz mit einer 3'-Hydroxylgruppe der Promotorsequenz PP veknüpft, wobei das 3'-Ende zwischen etwa Nukleotid -9 und dem 5'-Ende der Promotorregion (für den Fall des T7-Promotors) lokalisiert ist. In diesem Fall ist eine zweite, von PP getrennte Promotorsequenz, die die nicht in PP enthaltenen Nukleotide des Promotors enthält, erforderlich. (Fall C von FIG 2).

In einer fünften Ausführungsform ist das 3'-Ende der Effektorsequenz mit einer 5'-Hydroxylgruppe eines Nukleotids in der Promotorsequenz PP verknüpft, wobei die Position stromabwärts von der Position -12 liegt. Für diesen Fall ist ebenfalls die Benutzung eines zweiten Promotoroligonukleotids, welches die nicht in PP enthaltenen Nukleotide des Promotors enthält, bevorzugt (Fall D von FIG 2).

In einer sechsten Ausführungsform (Fall E von FIG 2) ist die Effektorsequenz PE direkt mit dem 5'-Ende der Promotorsequenz PP verknüpft.

Weitere Möglichkeiten sind im Fall F bis I von FIG 2 gezeigt.

Die bisher angegebenen Ausführungsformen können nun in analoger Weise auf den codierenden Strang des Promotors, die Promotorsequenz PP', übertragen werden, wobei jedoch die oben genannten Toleranzgrenzen der Promotorsequenz PP' zu berücksichtigen sind.

In einer ersten Ausführungsform (Fall K in FIG 2) ist die Effektorsequenz PE an die 3'-Hydroxylgruppe des codierenden Stranges der Promotorregion PP' geknüpft.

In einer zweiten Ausführungsform (Fall L in FIG 2) ist die Effektorsequenz PE so an ein 3'-Ende von PP' verknüpft, daß nur die Nukleotide -1 bis etwa -14 oder mehr der Sequenz PP' vorhanden sind. In diesem Fall ist kein Ergänzungsoligonukleotid für die einzelsträngigen Bereiche erforderlich.

In einer dritten Ausführungsform (Fall M in FIG 2) ist die Effektorsequenz mit ihrem 5'-Ende an das 3'-Ende des codierenden Stranges der Promotorregion so verknüpft, daß der die Effektorsequenz beinhaltende Strang der Promotorregion nur die Nukleotide zwischen Position -1 und Position etwa -12 enthält. In diesem Fall ist die Benutzung eines weiteren Oligonukleotids erforderlich.

In einer vierten Ausführungsform (N) ist das 3'-Ende der Effektorsequenz an das 5'-Ende des Stranges PP' innerhalb des Promotors gebunden. Auch hier empfiehlt es sich, die einzelsträngigen Bereiche durch Hybridisierung mit einem Oligonukleotid bzw. der verlängerten Templat-Nukleinsäuresequenz abzudecken.

In einer fünften Ausführungsform O ist ein 5'-Ende des einen Stranges mit dem 3'-Ende der Effektorsequenz so verbunden, daß der codierende Strang, verknüpft mit der Targetsequenz, die vollständige Promotorsequenz überspannt.

In einer sechsten Ausführungsform ist die Effektorsequenz am 3'-Ende der Templat-Nukleinsäuresequenz so gebunden, daß der codierende Strang des Promotors vollständig erhalten bleibt und nicht an Templatsequenz gebunden ist.

Bisher konnte eine Ausführungsform, bei der das 3'-Ende der Effektorsequenz an das 5'-Ende der Promotorregion an Strang PP' geknüpft ist, nicht zur Transkription verwendet werden.

In jedem dieser Fälle ist auch die Möglichkeit gegeben, daß das 5'-Ende von PP bzw. des es ergänzenden Oligonukleotids mit dem 3'-Ende von PP' oder des es ergänzenden Oligonukleotids direkt oder über Linker über eine Loop-Struktur verbunden ist. Bevorzugt sind die linken Enden jedes Falles in FIG 2 über einen Linker von 5 bis 40 Nukleotiden verknüpft.

Das erfindungsgemäße Transkriptionsverfahren erfordert die allgemein für Transkriptionsreaktionen üblichen Reaktionsbedingungen. Dazu gehört insbesondere eine DNA-abhängige RNA-Polymerase (Transkriptase). Diese synthetisiert eine Vielzahl von RNA-Kopien der Templat-Nukleinsäuresequenz TN, egal, ob diese Sequenz einzelsträngig oder in doppelsträngiger Form, z. B. in einer größeren Templat-Nukleinsäure, vorliegt. Die RNA-Moleküle werden in 3'-Richtung, ausgehend von der Startposition des Promotors, gebildet. Die Promotorsequenz wird nicht übersetzt. Die Transkriptase muß auf den verwendeten Promotor abgestimmt sein. Wird daher ein typischer Promotor des Bakteriophagen T7 verwendet, muß auch T7-RNA-Polymerase eingesetzt werden.

Weiterer unerläßlicher Bestandteil einer Transkriptionsreaktion sind Monoribonukleosid-Triphosphate (rNTP). Sie werden von der Polymerase an die entstehende Ribonukleinsäure angehängt. Diese rNTPs können unmodifiziert, jedoch auch modifiziert sein, z. B. durch Anhängen von Markierungsgruppen.

Eine Markierung im Sinne der vorliegenden Erfindung besteht aus einer direkt oder indirekt nachweisbaren Gruppe Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin-, Biotin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

In einer ersten, besonders einfachen Ausführungsform einer Transkriptionsreaktion zur Erzeugung von Ribonukleinsäuren aus einer einzelsträngigen oder einzelsträngig gemachten Templat-Nukleinsäure wird diese Templat-Nukleinsäure T mit einer Promotornukleinsäure P, die Teile Promotorsequenz PP, mit der Templat-Nukleinsäuresequenz TN hybridisierbare Sequenz PT und Effektor PE enthält, unter Hybridisierungs-Bedingungen in Kontakt gebracht. Diese Bedingungen können schon so gestaltet sein, daß die Transkriptionsreaktion nach Bildung des Hybrids ablaufen kann. Es ist jedoch auch möglich, die Bedingungen erst nach der Hybridisierung herzustellen, um die Transkriptionsreaktion zu starten. Die zur Transkription notwendigen Sequenzen PP' sind also in T enthalten oder werden zugegeben. Wenn ausreichend lange abgewartet wird, bildet sich eine große Anzahl von Transkripten. Diese Transkripte können Gegenstand jeder beliebigen Weiterbearbeitung oder Verwendung sein.

Eine Möglichkeit der Weiterbearbeitung ist die zyklische Amplifikation dieser Ribonukleinsäuren. Hierzu kann die Ribonukleinsäure mit einem Primer PR hybridisiert werden, dessen Sequenz so gewählt ist, daß seine Verlängerung zu einer Nukleinsäure führt, die einen Teil enthält, der wiederum mit dem Teil PT der Promotornukleinsäure P hybridisieren kann. Für diese Reaktion sind die für eine übliche cDNA-Synthese erforderlichen Reagenzien vonnöten, z. B. eine reverse Transkriptase und die Desoxyribonukleosid-Triphosphate (dNTP). Um eine Amplifikation zu erreichen, müssen die Ribonukleinsäure und das Verlängerungsprodukt von PR entweder voneinander getrennt oder die Ribonukleinsäure abgebaut werden. Die zweite Möglichkeit, insbesondere bei Verwendung von RNAse H, ist besonders vorteilhaft, da sie eine isotherme zyklische Reaktion ermöglicht. Das Verlängerungsprodukt des Primers kann, nachdem es einzelsträngig vorliegt, mit der Promotornukleinsäure hybridisieren. Unter den Reaktionsbedingungen kann daraufhin eine Verlängerung des 3'-Endes des Verlängerungsprodukts um den codierenden Strang PP' stattfinden. Die somit gebildete partiell doppelsträngige Nukleinsäure kann erneut zur Transkription zur Bildung von RNA verwendet werden. Mit der Anzahl der durchgeführten Reaktionszyklen erhöht sich sowohl die Anzahl der Verlängerungsprodukte als auch der im Reaktionsgemisch vorliegenden Menge an Ribonukleinsäure.

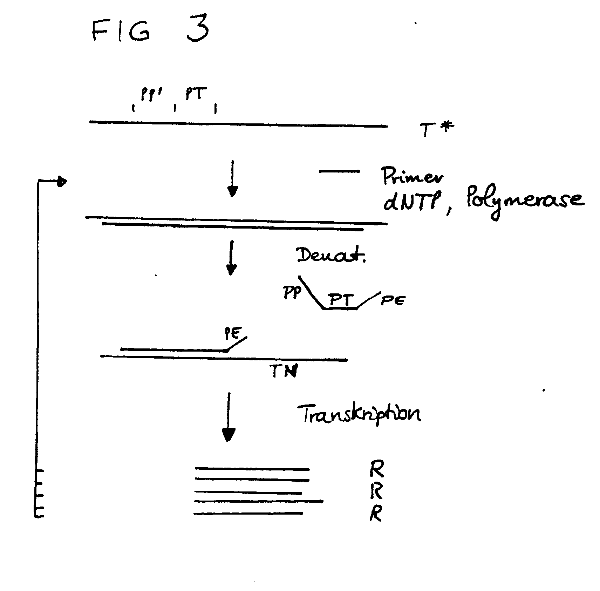

In einer in FIG 3 gezeigten weiteren Ausführungsform des erfindungsgemäßen Verfahrens wird der partiell doppelsträngige Nukleinsäurekomplex in einer Vorreaktion gebildet. Eine einzelsträngige Templat-Nukleinsäure T*, die eine Vorstufe der in die Transkriptionsreaktion eingesetzten Templat-Nukleinsäure darstellt, die in dieser Ausführungsform sowohl DNA als auch RNA darstellen kann, wird mit einem Primer hybridisiert. Der Primer wird unter Verwendung von dNTPs und einem von der Art der Templat-Nukleinsäure abhängigen Polymeraseenzym zu einem Verlängerungsprodukt verlängert, welches dann als Templat-Nukleinsäure dienen kann. Hier muß darauf geachtet werden, daß der Primer so gewählt wird, daß sein Verlängerungsprodukt die Templat-Nukleinsäuresequenz TN ist Es muß auch eine mit der Sequenz PT hybridisierbare Sequenz enthalten. PT ist nicht notwendig, wenn PP' in T* enthalten ist. Es wird nun dafür gesorgt, daß das Verlängerungsprodukt des Primers in einzelsträngiger Form vorliegt Dies kann entweder durch Denaturierung (insbesondere im Falle von DNA als Templat-Nukleinsäure T*) oder durch Behandlung mit RNase H (für den Fall,daß die Templat-Nukleinsäure T* RNA ist) geschehen. Anschließend wird das Verlängerungsprodukt des Primers mit einer Promotornukleinsäure P hybridisiert, die in dieser Reihenfolge die Komponenten PP', PT und PE enthält. Anschließend kann die Transkriptionsreaktion ablaufen. Auch hier ist es möglich, die gebildete RNA zyklisch zu amplifizieren, wobei wiederum der Primer und die Promotornukleinsäure P abwechselnd Einsatz finden. Die gebildete RNA wird dann erneut als Templat-Nukleinsäure T* eingesetzt.

Im Stand der Technik existieren eine ganze Reihe von Amplifikationsverfahren, die unter Verwendung von Promotornukleinsäuren ablaufen. Bei all diesen Verfahren stellt sich das Problem der Bildung von abortiven Transkripten. Diese Verfahren können erfindungsgemäß dadurch verbessert werden, daß als Promotomukleinsäuren die erfindungsgemäßen Promotornukleinsäuren eingesetzt werden. Beispiele für die bekannten Verfahren sind beschrieben in EP-A-0408295, EP-A-0329822, WO 90/01068, EP-A-0373960, EP-A-0397269 und EP-A-0369775.

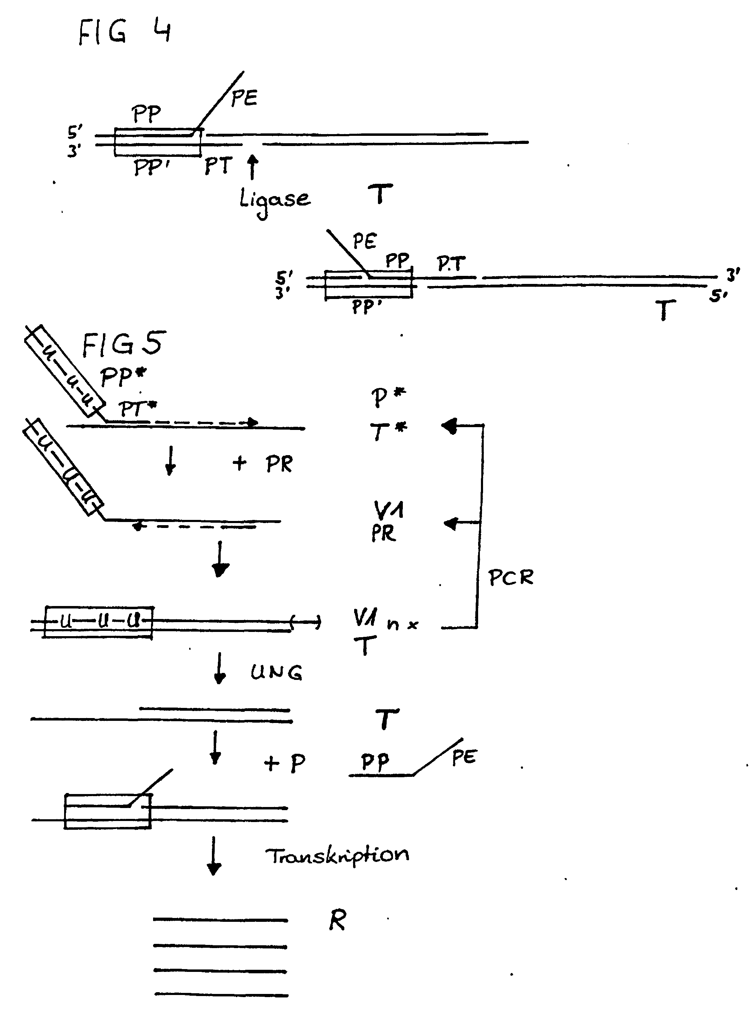

In einer dritten Ausführungsform (FIG 4) dient eine partiell doppelsträngige Nukleinsäure, z. B. DNA, die einen einzelsträngigen Bereich aufweist, als Templat-Nukleinsäure T. Diese Art von Templat-Nukleinsäuren sind beispielsweise Fragmente doppelsträngiger Nukleinsäure, die mittels Verdau durch Restriktionsenzyme unter Erzeugung von überhängenden Enden entstehen. Ein solches überhängendes Ende der Templat-Nukleinsäure dient zur Hybridisierung mit dem templatspezifischen Bereich PT einer partiell doppelsträngigen Promotornukleinsäure P. Die Promotornukleinsäure enthält in einem ersten Strang die Promotorsequenz PP, woran, direkt oder über einen Linker am 3'-Ende oder innerhalb der Promotorsequenz die Effektorsequenz gebunden ist. Außerdem enthält die Promotornukleinsäure P auf einem anderen Strang die Promotorsequenz PP' und, in 5'-Richtung die templatspezifische Sequenz PT. Unter Hybridisierungsbedingungen bildet sich ein Komplex aus Promotornukleinsäure und Templat-Nukleinsäure. Gewünschtenfalls kann das 5'-Ende der templatspezifischen Sequenz mit in der Nähe befindlichem 3'-Ende der Templat-Nukleinsäure mittels einer Ligase kovalent verknüpft werden. Danach steht der Nukleinsäurekomplex zur Bildung von Transkripten zur Verfügung. Analog kann die Sequenz PT am 3'-Ende von PP lokalisiert sein und PT ist komplementär zu Teilen von TN oder zu TN.

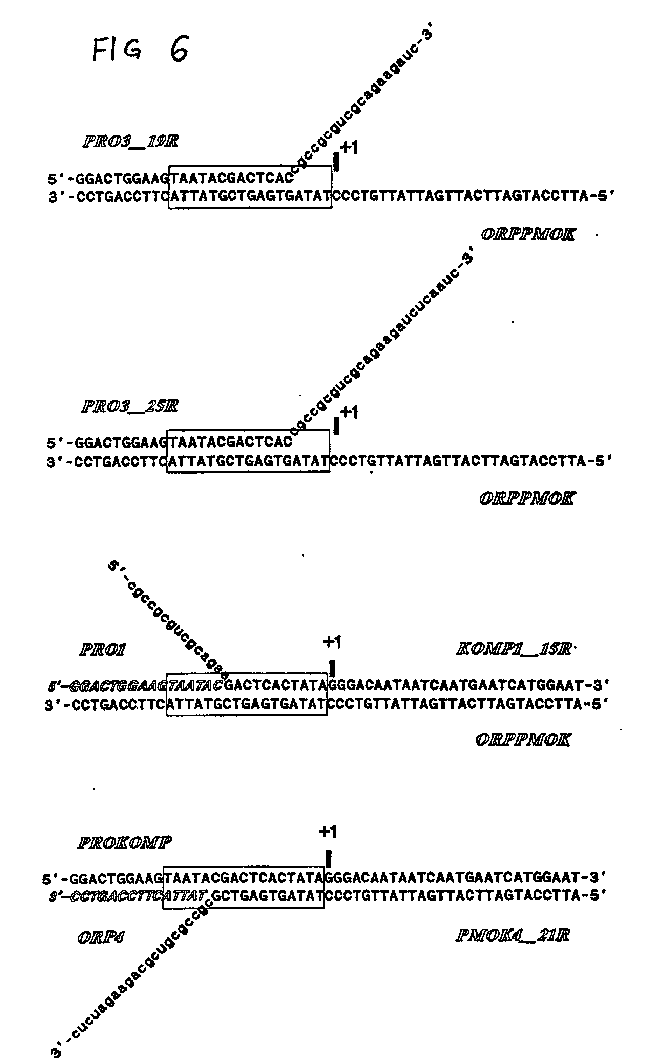

In einer vierten Ausführungsform (FIG 5) wird ein ganz ähnlicher Nukleinsäurekomplex ohne Notwendigkeit einer Restriktionsenzymspaltung hergestellt. Eine beliebige, einzelsträngige oder einzelsträngig gemachte Templat-Nukleinsäure T wird mit einer ersten Promotornukleinsäure P*, die eine abbaubare Promotorsequenz PP*, in der z. B. Thyminbasen mindestens teilweise durch Uracilbasen ersetzt sind, und am 3'-Ende der Promotorsequenz eine erste Primersequenz PT*, die zu der Templat-Nukleinsäure komplementär ist, umgesetzt. Unter Bedingungen, unter denen die Polymerasekettenreaktion (EP-A-0202184) abläuft, d. h. unter Verwendung eines weiteren Primers PR, der zu einem Teil des mit Hilfe des ersten Primers gebildeten Verlängerungsproduktes V1 komplementär ist, wird eine doppelsträngige Nukleinsäure gebildet, die an ihrem einen Ende die doppelsträngige Promotorsequenz und am anderen Ende die doppelsträngige Sequenz des zweiten Primers enthält. Das Verlängerungsprodukt des Primers PR dient später als Templat-Nukleinsäure T. Durch Behandlung mit Uracil-N-Glycosilase wird die Promotorsequenz PP* abgebaut. Die nun im Promotorteil mindestens teilweise einzelsträngige Nukleinsäure wird mit einer erfindungsgemäßen teilweise oder vollständig einzelsträngigen Promotornukleinsäure P hybridisiert, die eine zu dem einzelsträngigen Bereich komplementäre Promotorsequenz PP und daran direkt gebunden eine Effektorsequenz PE enthält. Der so gebildete Nukleinsäurekomplex steht ebenfalls wieder zur Transkription zur Verfügung.

Ebenfalls Gegenstand der Erfindung sind Promotornukleinsäuren, die eine Promotorsequenz PP oder/und deren Gegenstrang PP' und eine Effektorsequenz PE, die nicht DNA ist, enthalten. Die Promotorsequenzen PP bzw. PP' unterscheiden sich von der Art der sie aufbauenden Bestandteile von der Effektorsequenz PE. Bevorzugt sind die Promotorsequenzen PP bzw. PP' DNA. Die Promotornukleinsäure ist bevorzugt ein einzelsträngiges Molekül, kann jedoch auch doppelsträngig sein. Im Falle der Doppelsträngigkeit enthält die Promotornukleinsäure sowohl PP als auch PP* bzw. Teile davon, so daß die daraus gebildete Promotorregion funktionell ist.

Wie in den Fällen A bis E und G (FIG 2) geschildert, liegen die Sequenzen PP und PE bevorzugt auf einem Strang. Sofern die Promotornukleinsäure auch eine templatspezifische Sequenz PT enthält, liegt auch diese bevorzugt auf dem gleichen Strang wie PP und PE bzw. PP' und PE (Fall G).

Analog dazu, in den Fällen K, L und N der FIG 2, liegen PP' und PE bevorzugt auf einem Strang.

Ebenfalls Gegenstand der Erfindung sind Reagenzkits, enthaltend die für die durchzuführende Transkriptionsreaktion notwendigen Reagenzien, mit Ausnahme der Templat-Nukleinsäure von der Ribonukleinsäuren hergestellt werden sollen, wobei mindestens eine Promotornukleinsäure P enthalten ist.

Ein weiterer Gegenstand der Erfindung ist die Verwendung von Überhängen, insbesondere aus RNA an Promotorsequenzen zur Verringerung der Entstehung abortiver Transkripte, zur Verbesserung der quantitativen Bestimmung von Nukleinsäureprodukten in Transkriptionsverfahren, zur Erhöhung der Sensitivität bei Nukleinsäurebestimmungen, bzw. zur Verminderung des Mononukleotidbedarfs in Transkriptionsreaktionen.

Durch die erfindungsgemäße Reduktion der gebildeten Menge von abortiven Transkripten werden die bekannten Transkriptionsverfahren deutlich verbessert. Insbesondere führen die Überhänge zur Verbesserung der quantitativen Bestimmung von Nukleinsäureprodukten in Transkriptionsverfahren. Eine solche Bestimmung war bisher deutlich erschwert, wenn man die gebildete Menge an abortiven Transkripten berücksichtigt, die durchaus in der Größenordnung von bis zu 80 % der überhaupt gebildeten Transkripte lag. Erfindungsgemäß kann das erfindungsgemäße Verfahren auch zur Erhöhung der Sensitivität bei Nukleinsäurebestimmungen in Transkriptionsverfahren benutzt werden, da die entstandenen abortiven Transkripte zu einem Hintergrundsignal für die Bestimmung der Transkripte voller Länge führten, welche nun verringert ist. Die Entstehung der abortiven Transkripte hatte außerdem einen erhöhten Bedarf von rNTPs zur Folge, die für die Entstehung der Transkripte voller Länge nicht mehr zur Verfügung standen. Die Erfindung führt dazu, daß weniger Ribonukleotide eingesetzt werden können. Es ist außerdem durchaus denkbar, daß die Menge an gebildeten Transkripten voller Länge bei gleichbleibender rNTP-Menge erhöht ist.

Im Rahmen der Erfindung wurde festgestellt, daß DNA-Überhänge anstelle der RNA-Überhänge die Folge haben, daß die Menge an insgesamt gebildeten Transkripten verringert oder sogar ganz unterbunden wird, in Abhängigkeit der Länge der DNA-Überhänge. Soweit dies erkennbar war, führte der vollständige Ersatz der erfindungsgemäßen Effektorsequenzen durch DNA-Überhänge in den meisten Fällen nicht zu den erfindungsgemäßen Effekten.

Wenngleich eine Ursache für den erfindungsgemäßen Effekt nicht mit Sicherheit angegeben werden kann, so scheint es doch denkbar, daß RNA-Polymerasen, wie die untersuchte T7-RNA-Polymerase, mittels eines RNA-Überhanges am oder im Promotorbereich von einer abortiven in eine prozessive Form überführt werden können.

FIG 6 zeigt die in den Beispielen benutzten Oligonukleotide und deren Sequenzen, wobei Desoxyribonukleotide mit Großbuchstaben und Ribonukleotide mit Kleinbuchstaben geschrieben sind. Alle Oligonukleotide sind linear und einzelsträngig.

Die folgende Beispiele sollen die Erfindung näher erläutern:

### Beispiel 1:

Durch Zusammenpipettieren der in Tabelle 1 angegebenen Komponenten wurden ein Reaktionsansatz A und ein Reaktionsansatz B hergestellt Ansatz A enthält alle Charakteristika von Ansatz B mit der Ausnahme der Effektorsequenz PE.

**Tabelle 1:**

| 20 µl | A(µl) | B (µl) |
|---|---|---|
| PRO319R (10 pmol/µl) (SEQ.ID.NO. 1) | - | 1 |
| PRO3 (10 pmol/µl) (SEQ.ID.NO. 2) | 1 | - |
| ORPPMOK (10 pmol/µl) (SEQ.ID.NO. 3) | 1 | 1 |
| 10 mM ATP, CTP, GTP, UTP | 2 | 2 |
| 10 x Transkriptionspuffer | 2 | 2 |
| 1% Triton | 0,5 | 0,5 |
| Rnasin (40 U/µl) | 0,5 | 0,5 |
| H₂O | 11 | 11 |
| T7-Pol (700 U/µl) | 1 | 1 |
| α-[³²P]-CTP | 1 | 1 |

| 10 x Transkriptionspuffer: | | |
|---|---|---|
| 400 | mM | Tris (pH 7,9) |
| 60 | mM | MgCl₂ |
| 100 | mM | DTT |
| 20 | mM | Spermidin. |

Die oben genannten Ansätze wurden bei 37 °C inkubiert. Nach 5, 10, 15, 30 und 60 Minuten wurden jeweils 4 µl Flüssigkeit entnommen und die Reaktion mit je 3 µl Auftragspuffer für ein Sequenzgel gestoppt

Zur Bestimmung der relativen Mengen an entstandenen Transkripten wurden diese in einem 20 %-Sequenzgel (nach Sambrook et aL (1989) Molecular Cloning, CSH Laboratory Press Seite 6.36 ff.) durchgeführt. Aufgetragen wurden je 3 µl nach Denaturierung. Die angelegte Spannung betrug 2300 Volt. Das Gel wurde autoradiographisch abgebildet (Figur 7). Im Gegensatz zu in EP-A-408295 abgebildeten Gelen ist auch der Bereich erfaßt, in dem die kurzen Transkripte sichtbar sind. Es ist klar erkennbar, daß die Menge an abortiven Transkripten im Ansatz B gegenüber Ansatz A stark verringert ist.

Das in diesem Beispiel beschriebene Vorgehen kann als generelle Arbeitsvorschrift für die Transkription unter Zuhilfenahme der erfindungsgemäßen Promotornukleinsäuren verwendet werden.

Dieses Beispiel zeigt, daß die Effektorsequenz PE an das 3'-Ende der Promotorsequenz PP geknüpft werden kann, selbst wenn dieses Ende innerhalb der Promotorregion liegt und der nicht-codierende Strang der Promotorsequenz nicht vollständig ist. Im vorliegenden Fall sind die Positionen -1 bis -4 des nicht-codierenden Stranges der Promotorregion nicht präsent, da das 5'-Ende der Effektorsequenz mit der 3'-Hydroxylgruppe des Nukleotids in Position -5 (Cytosin) verknüpft ist.

### Beispiel 2:

Nach der Verfahrensvorschrift von Beispiel 1 wurde die Erfindung an weiteren Ausführungsmöglichkeiten überprüft.
a) Statt der in Beispiel 1 genannten Nukleinsäuren wurde in einer Ausführungsform, die der oben genannten Ausführungsmöglichkeit 5 (Fall D von FIG 2) entspricht, die Effektorsequenz am 5'-Ende einer Promotorsequenz PP innerhalb der Promotorregion gebunden. Als Promotorregion wird die Consensussequenz der T7-RNA-Polymerase Promotoren verstanden. Die dadurch entstehende Promotornukleinsäure P wird KOMP1_15R (SEQ.ID.NO. 4)genannt. Diese Promotornukleinsäure enthält die Positionen -1 bis -11 der Promotorregion von T7. Darüber hinaus wurde der Reaktionsmischung ein weiteres Oligonukleotid mit dem Namen PRO1 (SEQ.ID.NO. 5) zugesetzt, welches die Positionen -12 bis -17 des nicht-codierenden Stranges der Promotorregion enthält, darüber hinaus jedoch noch weitere Nukleotide, die komplementär zur Templat-Nukleinsäure T sind. Als Templat-Nukleinsäure wurde ein Oligonukleotid namens ORPPMOK (SEQ.ID.NO. 3) eingesetzt, welches eine Templat-Nukleinsäuresequenz, eine Promotorsequenz PP' und einen weiteren, zu dem Oligonukleotid PRO1 (SEQ.ID.NO. 5) komplementären Bereich enthält.
b) In einer weiteren Ausführungsform wurde gezeigt,daß es auch möglich ist, die Effektorsequenz PE an eine Position innerhalb des codierenden Stranges der Promotorregion zu binden. Im vorliegenden Fall wurde PE an Guanosin in Position -12 der Promotorregion gebunden. Die damit gebildete Promotornukleinsäure enthält vom 3'-Ende her gesehen die Effektorsequenz PE, eine Promotorsequenz PP' sowie eine Templat-Nukleinsäure TN. Diese Promotornukleinsäure wurde mit dem Namen PMOK4_21R (SEQ.ID.NO. 6) benannt. Außerdem wird der Reaktionsmischung ein Oligonukleotid ORP4 zugegeben, welches an seinem 5'-Ende die restlichen 5 Nukleotide des codierenden Stranges der Promotorregion aufweist und darüber hinaus noch weitere Nukleotide, die zu einem PROKOMP (SEQ.ID.NO. 7) genannten Strang komplementär sind. Das Oligonukleotid PROKOMP, welches vom 5'-Ende her gesehen eine Sequenz komplementär zum 3'-Ende von ORP4 (SEQ.ID.NO. 8), dann den nicht-codierenden Strang des Promotors und anschließend eine zu der Templat-Nukleinsäure komplementäre Sequenz enthält wird zugesetzt um die erstgenannten Oligonukleotide zusammenzuhalten und die Promotorregion funktionell zu machen.
c) In einem weiteren Beispiel, welches von der Auswahl der Templat-Nukleinsäure und der Promotorsequenz PP Beispiel 1 entspricht, wurde der Einfluß der Länge der Effektorsequenz getestet. Während im Beispiel 1 Effektorsequenz 19 Ribonukleotide lang ist, enthält PRO3_25R (SEQ.ID.NO. 9) eine Effektorsequenz der Länge 25 Ribonukleotid-Einheiten. Damit wurde gezeigt, daß die Länge der Effektorsequenz, zumindest in den gezeigten Bereichen, keinen wesentlichen Einfluß auf den erfindungsgemäßen Effekt hat.
   In Figur 6 sind die Ribonukleotide in Kleinbuchstaben, die Desoxyribonukleotide in Großbuchstaben angegeben. Die Konsensus-Sequenz des Promotors wurde durch einen Rahmen und der Transkriptionsstart mit +1 gekennzeichnet.

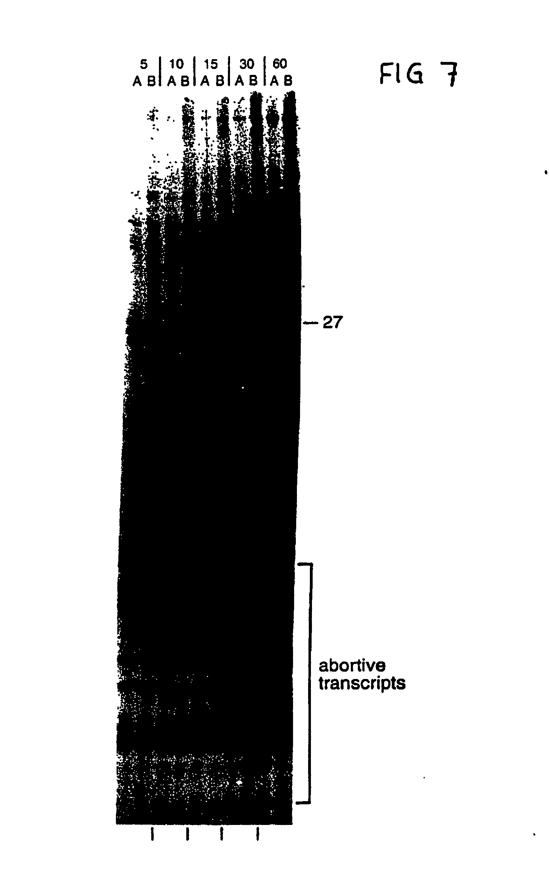

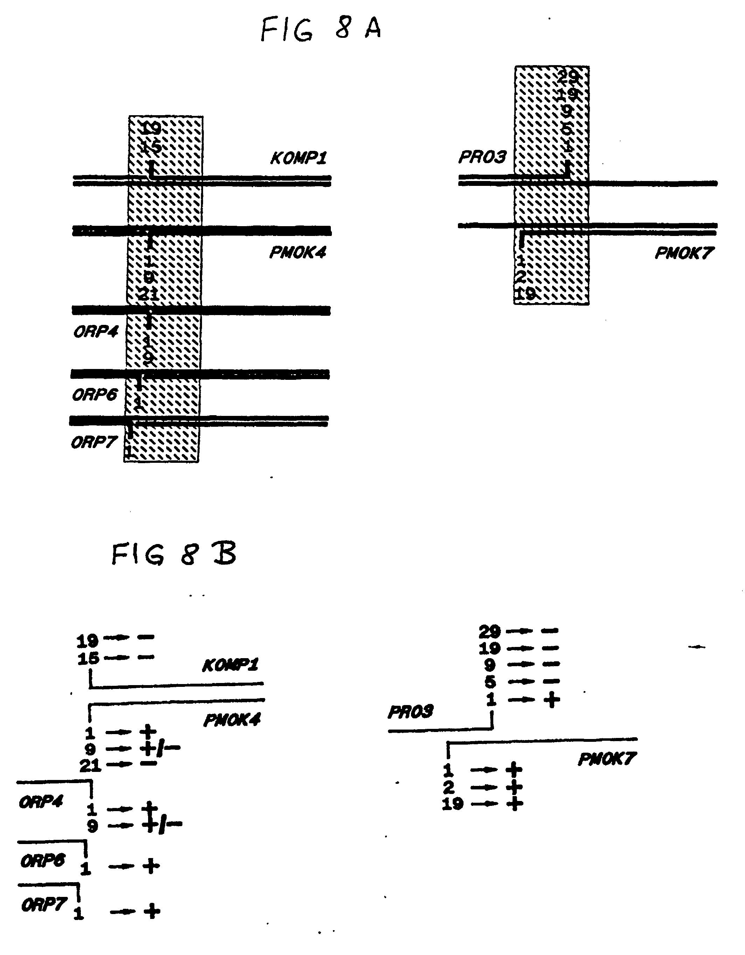

   In Figur 8B ist schematisch gezeigt, welchen Einfluß die Position und Länge von DNA-Überhängen im Promotorbereich hat. Die schraffierten Bereiche (FIG 8A) kennzeichnen die Promotorregion. Die Zahlen an den senkrechten Linien geben die jeweilige Anzahl der den Überhang bildenden Nukleotide an. "+" bedeutet, daß eine effiziente Transkription stattfand, "-" heißt, daß die Transkription durch den DNA-Überhang vollständig inhibiert wurde, "+/-" steht für reduzierte Effizienz (FIG 8B).

### Beispiel 3:

In einem weiteren Beispiel wurde untersucht, welchen Einfluß ein Schnitt (Nick) in der Promotorregion hat. In Figur 1 ist gezeigt, ob ein Nick und Fehlen der Phosphatgruppe zwischen den angegebenen Nukleotidpositionen im codierenden bzw. nicht-codierenden Strang der Promotorregion toleriert (+) oder nicht toleriert (-) wurde (FIG 1).

### SEQUENZPROTOKOLL

### (1) ALLGEMEINE ANGABEN:

(i) ANMELDER:
   (A) NAME: Boehringer Mannheim GmbH
   (B) STRASSE: Sandhoferstr. 116
   (C) ORT: Mannheim
   (E) LAND: DE
   (F) POSTLEITZAHL: 68305
   (G) TELEFON: 0621 759 4348
   (H) TELEFAX: 0621759 4457
(ii) BEZEICHNUNG DER ERFINDUNG: Verbessertes Transkriptionsverfahren
(iii) ANZAHL DER SEQUENZEN: 10
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30 (EPA)

### (2) ANGABEN ZU SEQ ID NO: 1:

(i) SEQUENZKENNZEICHEN:
   (A) LÄNGE: 42 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligonukleotid"
(iii) HYPOTHETISCH: NEIN
(ix) MERKMAL:
   (A) NAME/SCHLÜSSEL: misc_feature
   (B)LAGE:1..23
   (D) SONSTIGE ANGABEN:/note= "DNA"
(ix) MERKMAL:
   (A) NAME/SCHLÜSSEL: misc_feature
   (B) LAGE:24..42
   (D) SONSTIGE ANGABEN:/note= "RNA"
(xi) SEQUENZBESCHREIBUNG: SEQ ID NO: 1:
   GGACTGGAAG TAATACGACT CACCGCCGCG UCGCAGAAGA UC 42

### (2) ANGABEN ZU SEQ ID NO: 2:

(i) SEQUENZKENNZEICHEN:
   (A) LÄNGE: 23 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligonukleotid"
(iii) HYPOTHETISCH: NEIN
(xi) SEQUENZBESCHREIBUNG: SEQ ID NO: 2:
   GGACTGGAAG TAATACGACT CAC 23

### (2) ANGABEN ZU SEQ ID NO: 3:

(i) SEQUENZKENNZEICHEN:
   (A) LÄNGE: 54 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligodeoxyribonukleotid"
(iii) HYPOTHETISCH: NEIN
(xi) SEQUENZBESCHREIBUNG: SEQ ID NO: 3:

### (2) ANGABEN ZU SEQ ID NO: 4:

(i) SEQUENZKENNZEICHEN:
   (A) LÄNGE: 53 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligonukleotid"
(iii) HYPOTHETISCH: NEIN
(ix) MERKMAL:
   (A) NAME/SCHLÜSSEL: misc_feature
   (B) LAGE:1.. 15
   (D) SONSTIGE ANGABEN:/note= "RNA"
(ix) MERKMAL:
   (A) NAME/SCHLÜSSEL: misc_feature
   (B) LAGE:16..53
   (D) SONSTIGE ANGABEN:/note= "DNA"
(xi) SEQUENZBESCHREIBUNG: SEQ ID NO: 4:

### (2) ANGABEN ZU SEQ ID NO: 5:

(i) SEQUENZKENNZEICHEN:
   (A) LÄNGE: 16 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligodeoxyribonukleotid"
(iii) HYPOTHETISCH: NEIN
(xi) SEQUENZBESCHREIBUNG: SEQ ID NO: 5:
   GGACTGGAAG TAATAC   16

### (2) ANGABEN ZU SEQ ID NO: 6:

(i) SEQUENZKENNZEICHEN:
   (A) LÄNGE: 60 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligonukleotid"
(iii) HYPOTHETISCH: NEIN
(ix) MERKMAL:
   (A) NAME/SCHLÜSSEL: misc_feature
   (B) LAGE:1..39
   (D) SONSTIGE ANGABEN:/note= "DNA"
(ix) MERKMAL:
   (A) NAME/SCHLÜSSEL; misc_feature
   (B) LAGE:40..60
   (D) SONSTIGE ANGABEN:/note= "RNA"
(xi) SEQUENZBESCHREIBUNG: SEQ ID NO: 6:

### (2) ANGABEN ZU SEQ ID NO: 7:

(i) SEQUENZKENNZEICHEN:
   (A) LÄNGE: 54 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligodeoxyribonukleotid"
(iii) HYPOTHETISCH: NEIN
(xi) SEQUENZBESCHREIBUNG: SEQ ID NO: 7:

### (2) ANGABEN ZU SEQ ID NO: 8:

(i) SEQUENZKENNZEICHEN:
   (A) LÄNGE: 15 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligodeoxyribonukleotid"
(iii) HYPOTHETISCH: NEIN
(xi) SEQUENZBESCHREIBUNG: SEQ ID NO: 8:
   TATTACTTCC AGTCC 15

### (2) ANGABEN ZU SEQ ID NO: 9:

(i) SEQUENZKENNZEICHEN:
   (A) LÄNGE: 48 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligonukleotid"
(iii) HYPOTHETISCH: NEIN
(ix) MERKMAL:
   (A) NAME/SCHLÜSSEL: misc_feature
   (B) LAGE:1..23
   (D) SONSTIGE ANGABEN:/note= "DNA"
(ix) MERKMAL:
   (A) NAME/SCHLÜSSEL: misc_feature
   (B) LAGE:24..48
   (D) SONSTIGE ANGABEN:/note= "RNA"
(xi) SEQUENZBESCHREIBUNG: SEQ ID NO: 9:

### (2) ANGABEN ZU SEQ ID NO: 10:

(i) SEQUENZKENNZEICHEN:
   (A) LÄNGE: 54 Basenpaare
   (B) ART: Nucleotid
   (C) STRANGFORM: Doppelstrang
   (D) TOPOLOGIE: linear
(ii) ART DES MOLEKÜLS: Sonstige Nucleinsäure
   (A) BESCHREIBUNG: /desc = "doppelstraengiges Oligodeoxyribonukleotid"
(iii) HYPOTHETISCH: JA
(xi) SEQUENZBESCHREIBUNG: SEQ ID NO: 10:

## Patentansprüche

1. Verfahren zur Transkription einer Templat-Nukleinsäuresequenz TN in einer Templat-Nukleinsäure T in Ribonukleinsäure R durch
a) Bildung eines partiell oder vollständig doppelsträngigen Nukleinsäurekomplexes, enthaltend
- eine funktionelle, zumindest partiell doppelsträngige Promotorregion aus Promotorsequenzen PP und PP' sowie eine Effektorsequenz PE, die weder mit der Templat-Nukleinsäuresequenz TN noch mit deren Gegenstrang TN', noch mit der Promotorsequenz PP noch deren Gegenstrang PP' unter den gegebenen Reaktionsbedingungen hybridisieren kann, wobei die Effektorsequenz PE RNA ist, und
- eine mit der Promotorregion funktionell verknüpfte Templat-Nukleinsäuresequenz TN
b) und Einstellung von Bedingungen, unter denen die Ribonukleinsäure R unter Verwendung von TN als Templat unter Kontrolle des Promotors gebildet wird.

2. Verfahren gemäß Anspruch 1, **dadurch gekennzeichnet, daß** die Effektorsequenz PE direkt oder über einen Linker an die Promotorregion gebunden ist.

3. Verfahren gemäß Anspruch 1 oder 2, **dadurch gekennzeichnet, daß** PE am Ende oder innerhalb von PP oder PP' gebunden ist.

4. Verfahren gemäß Anspruch 1 oder 2 **dadurch gekennzeichnet, daß** der Nukleinsäurekomplex dadurch gebildet wird, daß eine Promotornukleinsäure P an die Templat-Nukleinsäure T hybridisiert wird, wobei die Promotornukleinsäure P folgende Teile enthält:
- eine Promotorsequenz PP oder/und deren Gegenstrang PP'
- eine mit zumindest einem Teil der Templat-Nukleinsäure hybridisierbare Sequenz PT und
- die Effektorsequenz PE.

5. Verfahren gemäß Anspruch 1 oder 2, **dadurch gekennzeichnet, daß** die Templat-Nukleinsäuresequenz TN oder TN' Teil einer Nukleinsäure mit einem einzelsträngigen und einem doppelsträngigen Bereich ist, wobei eine Sequenz PT mit dem einzelsträngigen Bereich der Templat-Nukleinsäure hybridisiert ist oder hybridisiert wird.

6. Verfahren gemäß Anspruch 1, 2 oder 3, **dadurch gekennzeichnet, daß** die Templat-Nukleinsäuresequenz TN mit der Promotorsequenz PP' kovalent verknüpft ist.

7. Verfahren gemäß Anspruch 1 oder 2, **dadurch gekennzeichnet, daß** die Effektorsequenz PE an dem 3'-Ende der Promotorsequenz PP gelegen ist.

8. Verfahren gemäß Anspruch 2, **dadurch gekennzeichnet, daß** die Effektorsequenz PE weder komplementär noch sequenzidentisch zu einem Teil der Templat-Nukleinsäure T ist.

9. Verfahren gemäß Anspruch 1 oder 2, **dadurch gekennzeichnet, daß** die Effektorsequenz PE mindestens 4 Nukleotide bevorzugt mindestens 9 lang ist.

10. Verfahren nach Anspruch 1 oder 2, **gekennzeichnet durch**
- Hybridisierung einer ersten einzelsträngigen Templat-Nukleinsäure T*, die eine Vorstufe der in die Transkriptionsreaktion eingesetzten Templat-Nukleinsäure darstellt, mit einer ersten Promotornukleinsäure P*, die eine mit Uracil-N-Glycosilase abbaubare Promotorsequenz PP* und eine mit der Templat-Nukleinsäure T* hybridisierbare Sequenz PT* enthält,
- Verlängerung der Promotornukleinsäure P* unter Verwendung der Templat-Nukleinsäure T* als Templat zu einem Verlängerungsprodukt V1,
- Bildung eines zu dem Verlängerungsprodukt V1 komplementären Stranges,
- Abbau der Promotorsequenz PP*,
- Verwendung des entstandenen komplementären Stranges als Templat-Nukleinsäure T.

11. Verfahren zum Nachweis von Nukleinsäuresequenzen, bei dem die nachzuweisenden Nukleinsäuresequenzen als Templat-Nukleinsäure T von Anspruch 1 oder 2 oder T* von Anspruch 10 verwendet werden und der Nachweis der Nukleinsäuresequenzen über die gebildete RNA geführt wird.

12. Promotornukleinsäure enthaltend eine Promotorsequenz PP oder deren Gegenstrang PP' und eine Effektorsequenz PE, die RNA ist.

13. Promotornukleinsäure gemäß Anspruch 12, **dadurch gekennzeichnet, daß** sie auch den jeweiligen Gegenstrang PP' bzw. PP enthält.

14. Promotornukleinsäure nach Anspruch 12, **dadurch gekennzeichnet, daß** sie außerdem einen templatspezifischen Teil PT enthält.

15. Promotornukleinsäure gemäß Anspruch 14, **dadurch gekennzeichnet, daß** die templatspezifische Sequenz auf dem gleichen Strang wie PP und PE liegt.

16. Promotornukleinsäure gemäß Anspruch 14, **dadurch gekennzeichnet, daß** PT auf dem Gegenstrang zu PP und PE liegt.

17. Verwendung von RNA-Überhängen einer Promotorregion zur Verringerung der Entstehung abortiver Transkripte.

18. Verwendung von RNA-Überhängen einer Promotorregion zur Verbesserung der quantitativen Bestimmung von Nukleinsäuren in Transkriptionsverfahren.

19. Verwendung von RNA-Überhängen einer Promotorregion zur Erhöhung der Sensitivität bei Nukleinsäurebestimmungen in Transkriptionsverfahren.

20. Verwendung von RNA-Überhängen einer Promotorregion zur Verminderung des Mononukleotidbedarfs in Transkriptionsreaktionen.

## Claims

1. Method for transcribing a template nucleic acid sequence TN in a template nucleic acid T into ribonucleic acid R by
a) forming a partially or completely double-stranded nucleic acid complex comprising
- a functional, at least partially double-stranded promoter region consisting of promoter sequences PP and PP' and an effector sequence PE which can hybridize under the given reaction conditions neither with the template nucleic acid sequence TN nor with its counterstrand TN' nor with a promoter sequence PP or its counterstrand PP', the effector sequence PE being RNA, and
- a template nucleic acid sequence TN which is functionally linked to the promoter region
b) and adjusting conditions under which the ribonucleic acid R is formed under the control of the promoter using TN as the template.

2. Method as claimed in claim 1, **characterized in that** the effector sequence PE is linked to the promoter region either directly or via a linker.

3. Method as claimed in claim 1 or 2, **characterized in that** PE is bound to the end of PP or PP' or within PP or PP'.

4. Method as claimed in claim 1 or 2, **characterized in that** the nucleic acid complex is formed by hybridizing a promoter nucleic acid P with the template nucleic acid T, wherein the promoter nucleic acid P contains the following components:
- a promoter sequence PP or/and its counterstrand PP'
- a sequence PT which can hybridize with at least a part of the template nucleic acid
and
- the effector sequence PE.

5. Method as claimed in claim 1 or 2, **characterized in that** the template nucleic acid sequence TN or TN' is part of a nucleic acid containing a single-stranded and a double-stranded region, and a sequence PT is hybridized or becomes hybridized with the single-stranded region of the template nucleic acid.

6. Method as claimed in claim 1, 2 or 3, **characterized in that** the template nucleic acid sequence TN is covalently linked to the promoter sequence PP'.

7. Method as claimed in claim 1 or 2, **characterized in that** the effector sequence PE is located at the 3' end of the promoter sequence PP.

8. Method as claimed in claim 2, **characterized in that** the effector sequence PE is neither complementary nor has an identical sequence to a part of the template nucleic acid T.

9. Method as claimed in claim 1 or 2, **characterized in that** the effector sequence PE has a length of at least 4 nucleotides and preferably at least 9.

10. Method as claimed in claim 1 or 2, **characterized in that**
- a first single-stranded template nucleic acid T* which is a precursor of the template nucleic acid used in the transcription reaction is hybridized with a first promoter nucleic acid P* which contains a promoter sequence PP* that can be degraded by uracil-N-glycosylase and a sequence PT* that can hybridize with the template nucleic acid T*,
- the promoter nucleic acid P* is extended using the template nucleic acid T* as a template to form an extension product V1,
- a strand that is complementary to the extension product V1 is formed,
- the promoter sequence PP* is degraded,
- the resulting complementary strand is used as the template nucleic acid T.

11. Method for detecting nucleic acid sequences, in which the nucleic acid sequences to be detected are used as the template nucleic acid T of claim 1 or 2 or T* of claim 10 and the nucleic acid sequences are detected by means of the RNA that is formed.

12. Promoter nucleic acid containing a promoter sequence PP or its counterstrand PP' and an effector sequence PE which is RNA.

13. Promoter nucleic acid as claimed in claim 12, **characterized in that** it also contains the respective counterstrand PP' or PP.

14. Promoter nucleic acid as claimed in claim 12, **characterized in that** it additionally contains a template-specific part PT.

15. Promoter nucleic acid as claimed in claim 12, **characterized in that** the template-specific sequence is on the same strand as PP and PE.

16. Promoter nucleic acid as claimed in claim 14, **characterized in that** PT is on the counterstrand to PP and PE.

17. Use of RNA overhangs of a promoter region to reduce the generation of abortive transcripts.

18. Use of RNA overhangs of a promoter region to improve the quantitative determination of nucleic acids in transcription methods.

19. Use of RNA overhangs of a promoter region to increase the sensitivity of nucleic acid determinations in transcription methods.

20. Use of RNA overhangs of a promoter region to reduce the mononucleotide requirement in transcription reactions.

## Revendications

1. Procédé pour la transcription en acide ribonucléique R d'une séquence matricielle d'acides nucléiques TN dans un acide nucléique matriciel T par
a) formation d'un complexe d'acides nucléiques partiellement ou complètement bicaténaire contenant
- une région de promoteur fonctionnelle, au moins partiellement bicaténaire constituée par des séquences de promoteur PP et PP' ainsi qu'une séquence effectrice PE, qui ne peut s'hybrider, ni avec la séquence matricielle d'acides nucléiques TN, ni avec le brin antagoniste TN' de cette demière, ni avec la séquence de promoteur PP, ni avec le brin antagoniste PP' de cette dernière dans les conditions réactionnelles indiquées, la séquence effectrice PE étant de l'ARN, et
- une séquence matricielle d'acides nucléiques TN liée de manière fonctionnelle à la région de promoteur
b) et réglage des conditions dans lesquelles l'acide ribonucléique R est formé sous le contrôle du promoteur en utilisant TN comme matrice.

2. Procédé selon la revendication 1, **caractérisé en ce que** la séquence effectrice PE est liée à la région de promoteur de manière directe ou via un lieur.

3. Procédé selon la revendication 1 ou 2, **caractérisé en ce que** PE est liée à l'extrémité ou à l'intérieur de PP ou de PP'.

4. Procédé selon la revendication 1 ou 2, **caractérisé en ce qu'**on forme le complexe d'acides nucléiques par hybridation d'un acide nucléique promoteur P à l'acide nucléique matriciel T, l'acide nucléique promoteur P contenant les parties indiquées ci-après :
- une séquence de promoteur PP et/ou son brin antagoniste PP'
- une séquence PT apte à s'hybrider à au moins une partie de l'acide nucléique matriciel, et
- la séquence effectrice PE.

5. Procédé selon la revendication 1 ou 2, **caractérisé en ce que** la séquence matricielle d'acides nucléiques TN ou TN' fait partie d'un acide nucléique comprenant une région monocaténaire et une région bicaténaire, dans lequel une séquence PT comprenant la région monocaténaire de l'acide nucléique matriciel a été soumise ou est soumise à une hybridation.

6. Procédé selon la revendication 1, 2 ou 3, **caractérisé en ce que** la séquence matricielle d'acides nucléiques TN est liée de manière covalente à la séquence de promoteur PP'.

7. Procédé selon la revendication 1 ou 2, **caractérisé en ce que** la séquence effectrice PE est disposée à l'extrémité 3' de la séquence de promoteur PP.

8. Procédé selon la revendication 2, **caractérisé en ce que** la séquence effectrice PE ne manifeste ni une complémentarité, ni une identité de séquence avec une partie de l'acide nucléique matriciel T.

9. Procédé selon la revendication 1 ou 2, **caractérisé en ce que** la séquence effectrice PE possède une longueur correspondant à au moins 4 nucléotides, de préférence à au moins 9 nucléotides.

10. Procédé selon la revendication 1 ou 2, **caractérisé par**
- l'hybridation d'un premier acide nucléique matriciel monocaténaire T*, qui représente un précurseur de l'acide nucléique matriciel mis en oeuvre dans la réaction de transcription, avec un premier acide nucléique promoteur P* qui contient une séquence de promoteur PP* apte à être dégradée à l'aide d'une uracil-N-glucosidase et une séquence PT* apte à s'hybrider avec l'acide nucléique matriciel T*,
- le prolongement de l'acide nucléique promoteur P* en utilisant l'acide nucléique matriciel T* à titre de matrice pour obtenir un produit de prolongement V1,
- formation d'un brin complémentaire au produit de prolongement V1,
- dégradation de la séquence de promoteur PP*,
- utilisation du brin complémentaire obtenu à titre d'acide nucléique matriciel T.

11. Procédé pour le décèlement de séquences d'acides nucléiques, dans lequel on utilise les séquences d'acides nucléiques à déceler à titre d'acide nucléique matriciel T selon la revendication 1 ou 2 ou T* selon la revendication 10 et on mène à bien le décèlement des séquences d'acides nucléiques via l'ARN formé.

12. Acide nucléique promoteur contenant une séquence de promoteur PP ou son brin antagoniste PP' et une séquence effectrice PE, qui représente de l'ARN.

13. Acide nucléique promoteur selon la revendication 12, **caractérisé en ce qu'**il contient également le brin antagoniste respectif PP' ou PP.

14. Acide nucléique promoteur selon la revendication 12, **caractérisé en ce qu'**il contient en outre une partie PT manifestant une spécificité matricielle.

15. Acide nucléique promoteur selon la revendication 14, **caractérisé en ce que** la séquence manifestant une spécificité matricielle est située sur le même brin que celui de PP et de PE.

16. Acide nucléique promoteur selon la revendication 14, **caractérisé en ce que** PT est situé sur le brin antagoniste par rapport à celui de PP et de PE.

17. Utilisation de chevauchements d'ARN d'une région de promoteur pour réduire la formation de transcrits abortifs.

18. Utilisation de chevauchements d'ARN d'une région de promoteur pour améliorer la détermination quantitative d'acides nucléiques dans des procédés de transcription.

19. Utilisation de chevauchements d'ARN d'une région de promoteur pour augmenter la sensibilité lors de déterminations d'acides nucléiques dans des procédés de transcription.

20. Utilisation de chevauchement d'ARN d'une région de promoteur pour diminuer le besoin en mononucléotides dans des réactions de transcription.
